# EUROPEAN PATENT APPLICATION

(11) **EP 1 629 781 A2**
(43) Date of publication of application: **01.03.2006**
(21) Application number: 05018512.3
(22) Date of filing: 25.08.2005
(51) Int. Cl.: A61B 17/06

(54) **Suture thread with needle**

(30) Priority: 27.08.2004 JP 2004248109
(71) Applicant: Mani, Inc., Shioya-gun, Tochigi-ken (JP)
(72) Inventor: Matsutani, Kanji, Shioya-gun Tochigi-ken (JP)
(74) Representative: Mussgnug, Bernd

(57) **Abstract**

When the eyeless needle and the suture thread are joined without forming a hole having a special shape in the eyeless needle configuring the suture thread with needle and using the suture thread made of multifilament thread, the step difference between the eyeless needle and the suture thread is reduced. The suture thread with needle A is configured by caulking and joining the multifilament thread 2 serving as the suture thread and the eyeless needle 1, and by removing one part of the fiber at the joined part of the multifilament thread 2 caulked and joined to the eyeless needle 1, the thickness of the reduced diameter part 2b becomes smaller than the thickness of the thread part 2a.

## Description

### BACKGROUND OF THE INVENTION

### 1. Field of the invention

The present invention relates to a suture thread with needle, configured by joining the suture thread to an eyeless needle, for piercing the body tissue with the eyeless needle and suturing the affected area or ligaturing the blood vessel with the suture thread, more specifically, relates to a suture thread with needle in which the difference between the thickness of the eyeless needle and the thickness of the suture thread is reduced.

### 2. Description of the Related Art

When suturing the delicate affected area or ligaturing the blood vessel, the suture thread with needle that employs the eyeless needle formed with a blind hole on the original end face and that joins the eyeless needle and the suture thread by inserting the end of the suture thread made of monofilament or multifilament into the blind hole of the eyeless needle and then caulking is used. The monofilament thread represented by nylon has a property of being hard to tie and easy to loosen but having a large tensile strength, whereas the multifilament represented by silk has a property of being easy to tie and hard to loosen but having a small tensile strength.

When performing suture or ligation using the suture thread with needle, the tissue may be damaged, or blood or body fluid may exude out from the hole formed when the affected area is pierced if a difference is created between the thickness of the eyeless needle and the thickness of the suture thread. Thus, a number of techniques have been developed and proposed for the purpose of realizing the suture thread with needle without a step difference between the eyeless needle and the suture thread.

For instance, in the technique disclosed in Japanese Utility-Model Application Publication (JP-Y) No. 60-41213, laser beam machining or turning machining is performed so that the thickness of the wall of the insertion hole formed in the eyeless needle is thinned from the back side towards the open side of the insertion hole. Thus, when the suture thread is inserted into the insertion hole and caulked (press to transform), the caulked part of the eyeless needle is slanted towards the suture thread side so that the diameter is reduced, thereby eliminating the step difference between the end of the eyeless needle and the suture thread.

In the technique disclosed in Japanese Patent Application Laid-Open (JP-A) No. 1-34625, the hole formed on the original end face of the eyeless needle is formed so that the diameter of the entrance part is made larger than the diameter of the back part through electric discharge machining. Thus, when the suture thread is inserted into the hole and caulked, a slant in which the diameter becomes smaller towards the original end face side is created at the caulked part, thereby reducing the step difference between the thickness at the original end face part of the eyeless needle and the thickness of the suture thread.

As noted above, any techniques disclosed in JP-Y No.60-41213 and JP-A No. 1-34625 both eliminate the step difference between the eyeless needle and the suture thread by defining the shape of the hole formed on the eyeless needle side so that when the suture needle is inserted into the relevant hole and then caulked, the shape of the caulked part is tapered towards the end face side.

On the other hand, in the technique disclosed in Japanese Patent Publication No. 2965667, the monofilament is heated and the heated part is extended to reduce the diameter, and further, the part having the reduced diameter is cut and used as the suture thread. By inserting the part with the reduced diameter of the monofilament into the hole formed in the eyeless needle and then caulking to join, the step difference between the diameter of the non-extended part of the monofilament and the diameter of the caulked part of the eyeless needle is reduced.

### SUMMARY OF THE INVENTION

In the techniques disclosed in JP-Y No.60-41213 and JP-A No. 1-34625, the hole in which the diameter becomes smaller from the end face side towards the back is machined at the end of the eyeless needle, or the outer periphery is machined so that the diameter becomes smaller towards the end face of the eyeless needle. However, when manufacturing the eyeless needle having an extremely small diameter such as used for delicate suture or ligation as seen in brain surgery and the like, the above mentioned machining becomes difficult to perform, and the manufacturing cost increases. Further, with the thinning of the wall thickness of the hole in an aim to eliminate the step difference, the holding force with respect to the suture thread becomes small.

In the technique of patent article 3, since the suture thread is limited to a material that is monofilament and that can be heated and extended such as, nylon, the technique cannot be applied to knitted thread or twisted thread made of multifilament, such as, silk and polyester.

The present invention aims to provide a suture thread with needle that reduces the step difference between the eyeless needle and the suture thread when the eyeless needle and the suture thread are joined without forming a hole of a special shape in the eyeless needle and using the suture thread made of multifilament thread.

In order to solve the above problems, the present invention proposes a suture thread with needle configured by caulking and joining a suture thread made of multifilament thread and an eyeless needle, where one part of the fiber at the joining part of the multifilament thread caulked and joined to the eyeless needle is removed so that the thickness of one part of the multifilament thread is smaller than the thickness of the other parts.

In the suture thread with needle according to the present invention, by removing one part of the fiber of the multifilament thread configuring the suture thread, the thickness of the removed part is made thinner than the thickness of the other parts. Therefore, when the thinned part where one part of the fiber of the multifilament thread is removed is inserted and caulked into the hole formed in the eyeless needle, the thick part of the multifilament thread faces the end of the eyeless needle thereby reducing the step difference with the relevant end.

In particular, the shape of the hole formed in the eyeless needle does not need to be defined, and thus wall thickness of the hole does not need to be thinned, and when the multifilament thread is inserted and caulked into the hole, the holding force with respect to the inserted multifilament thread is secured.

### BRIEF DESCRIPTION OF THE DRAWINGS

The invention, together with objects and advantages thereof, may best be understood by reference to the following description of the presently preferred embodiments together with the accompanying drawings in which:
Fig. 1 is a view explaining the configuration of a suture thread with needle A;
Fig. 2A is a view explaining the procedures for removing one part of fiber from the multifilament thread;
Fig. 2B is a view explaining the procedures for removing one part of fiber from the multifilament thread;
Fig. 2C is a view explaining the procedures for removing one part of fiber from the multifilament thread;
Fig. 2D is a view explaining the procedures for removing one part of fiber from the multifilament thread;
Fig. 3A is a view explaining the procedures for joining the multifilament thread in which one part of the fiber is removed with the hole of the eyeless needle;
Fig. 3B is a view explaining the procedures for joining the multifilament thread in which one part of the fiber is removed with the hole of the eyeless needle;
Fig. 3C is a view explaining the procedures for joining the multifilament thread in which one part of the fiber is removed with the hole of the eyeless needle;
Fig. 3D is a view explaining the procedures for joining the multifilament thread in which one part of the fiber is removed with the hole of the eyeless needle;
Fig. 4 is a view explaining the procedures for removing one part of fiber from the multifilament thread;
Fig. 4A is a view explaining the procedures for removing one part of fiber from the multifilament thread;
Fig. 4B is a view explaining the procedures for removing one part of fiber from the multifilament thread;
Fig. 4C is a view explaining the procedures for removing one part of fiber from the multifilament thread; Fig. 4D is a view explaining the procedures for removing one part of fiber from the multifilament thread;
Fig. 5A is a view explaining the procedures for joining the multifilament thread in which one part of the fiber is removed with the hole of the eyeless needle.
Fig. 5B is a view explaining the procedures for joining the multifilament thread in which one part of the fiber is removed with the hole of the eyeless needle.
Fig. 5C is a view explaining the procedures for joining the multifilament thread in which one part of the fiber is removed with the hole of the eyeless needle.
Fig. 5D is a view explaining the procedures for joining the multifilament thread in which one part of the fiber is removed with the hole of the eyeless needle.

### DESCRIPTION OF THE PREFERRED EMBODIMENTS

The most preferred embodiments of the suture thread with needle according to the present invention will now be explained. The suture thread with needle according to the present invention is configured by joining the eyeless needle, a needle that pierces through the body tissue and formed with a blind hole from the end face of the end on one side, and a multifilament thread made by knitting or twisting fiber such as silk, polyester and the like. The center of axle of the eyeless needle and the center of axle of the multifilament are coincided, and the difference between the thickness of the eyeless needle and the thickness of the multifilament thread is reduced or substantially diminished thereby preventing damage of the body tissue and preventing exudation of blood or body fluid from the body tissue.

In particular, when diminishing the difference between the thickness of the eyeless needle and the thickness of the multifilament thread, one part of the fiber of the multifilament thread is removed to partially reduce the thickness. When the part of reduced thickness is inserted into the hole formed in the eyeless needle and then caulked (press to transform), the multifilament of large thickness is joined to the eyeless needle. Thus, the difference between the thicknesses of the eyeless needle and the multifilament is reduced or substantially diminished.

The eyeless needle has a function of opening up or pushing the body tissue open at the site to be sutured or ligatured and passing the suture thread. Any needle having such function can be used. The general eyeless needle has a needlepoint part formed at the distal end part, a middle part formed continuously from the needlepoint part, from which middle part the other end side is continuously formed to become the original end including the caulked part, and a hole through which the suture thread is inserted along the center of axle (longitudinal direction of eyeless needle) formed at the end face (original end face) of the original end. The hole has the depth set in accordance with the thickness of the suture thread, and the part corresponding to the depth of the hole at the original end becomes the caulked part.

The eyeless needle is set with a specification including various outer shapes or cross sectional shapes, thickness and the like in accordance with the body tissue to be applied, and the shape, dimension and the like are not uniquely limited. For instance, there exists an eyeless needle having a middle part in which the cross section that forms a sharp cutting blade from the sharp needlepoint part is a polygon shape, an eyeless needle having a middle part in which the cross section is circular from the blunt needlepoint part, an eyeless needle having a curved shape from the needlepoint part to the middle part, a eyeless needle formed linear from the needlepoint part to the middle part and the like.

The thickness (outer diameter of the original end) of the eyeless needle is set to be 0.05 mm for the narrowest ones and 1.78mm for the thickest ones, and the eyeglass needles of various sizes are prepared within such range at an interval of 0.02 mm or interval of 0.03 mm. Similarly, the diameter of the hole formed at the original end face of the eyeless needle is also set in correspondence to the thickness of the eyeless needle. When, for example, the thickness of the eyeless needle is 0.43 mm, the hole diameter is set to be 0.26 mm, and the depth is set to be 1.1 mm or greater.

The material of the eyeless needle is not particularly limited, and may be of any type as long the strength that can act against the piercing resistance when the body tissue is pierced and the opening performance necessary to reduce the piercing resistance are secured. Such material includes steel represented by carbon tool steels, stainless steel and the like, and can selectively use such materials. In particular, the austenite stainless steel that does not cause rust in the distributing process is preferably used. In case of the austenite stainless steel, since hardness through heat treatment cannot be expected, the material of the austenite stainless steel is desirably hardened by cool working.

The machining method of when forming the hole at the original end face of the eyeless needle is not particularly limited, and machining methods such as, a laser machining method of forming the hole by irradiating the laser light on the original end face or drilling method, or electric discharge machining or electron beam method and the like is selectively used.

The multifilament threads sutures or ligatures the body tissue of the affected area and is formed by knitting or twisting a great number of extremely thin fibers. The material of the multifilament thread only needs to be such that does not adversely affect the living body, and silk, polyester or nylon and the like is preferably used. However, in the present invention, the material is not limited and any multifilament thread which can be used as suture thread may be used.

The multifilament thread has the size defined as 1 to 5, 1 to 0 to 11 to 0 and the like. For example, size 1 has the thread diameter of 0.5 mm, size 5 has the thread diameter of 0.8 mm, size 1 to 0 has the thread diameter of 0.4 mm, and size 11 to 0 has the thread diameter of 0.019 mm. Therefore, the multifilament thread of various sizes is provided.

By selecting and joining a thicker multifilament thread in correspondence to the thickness or the hole diameter of the eyeless needle, the step difference between the eyeless needle and the multifilament thread is reduced.

When removing the fiber of one part of the multifilament thread, the part or the length to be removed is not limited, and the fiber equivalent to the length (sufficiently longer than the depth of the hole) corresponding to the depth of the hole formed in the eyeless needle along the center of axle (in the longitudinal direction) of the multifilament thread is removed. For instance, when the multifilament thread is a knitted thread or a twisted thread including the thread to become the core (core thread), the core thread may be pulled out, cut and then removed, or, irrespective of the presence of the core thread, the knitted fiber or the twisted fiber may be pulled out, cut and removed.

The removing amount of the fiber with respect to the thickness direction of the multifilament is not particularly limited, but the removing amount that can reduce the size of the thread diameter by about 1 rank to 3 ranks is preferable. For instance, one part of the fiber of the multifilament thread of size 3 to 0 (thread diameter of 0.25 mm) is removed to have the thickness of the removed part of preferably about size 4 to 0 (thread diameter of 0.2 mm) or size 5 to 0 (thread diameter of 0.5 mm).

As noted above, by removing the fiber of one part of the multifilament thread of size 3 to 0 and the like to have the thickness of the removed part similar to size 4 to 0, the multifilament thread of size 3 to 0 can be used in the same way as the multifilament thread of size 4 to 0.

Therefore, where the multifilament thread of size 4 to 0 is normally used, the multifilament thread of size 3 to 0 may be used for the eyeless needle having a thickness of 0.43 mm or the like., and as a result, the thickness of the multifilament thread is increased from 0.2 mm to 0.25 mm. Thus, the difference between the needle diameter and the thread diameter is reduced by 0.05 mm, which is the increase in the thickness of the multifilament thread.

When the fiber of one part of the multifilament is removed, the thread can be inserted into the hole formed at the original end face of the eyeless needle with the removed part as it is and then caulked. However, to easily perform the inserting task, an adhesive that does not adversely affect the living body is preferably applied and hardened at the part where the fiber is removed. In particular, by applying and hardening the adhesive to the part where the fiber is removed with the pull force applied thereon, the thickness of the removed site can be reasonably maintained.

When inserting the part where the fiber of the multifilament thread is removed into the hole formed at the original end face of the eyeless needle and then caulked, the tool to be used therefor is not limited. However, caulking is preferably performed in one step using a pair of molded dies configured so as to relatively move away from or move towards each other.

### [Embodiment 1]

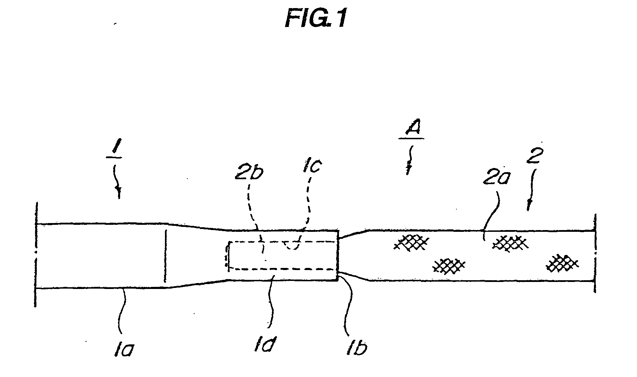

The configuration of the suture thread with needle according to the present invention will now be explained using the figures. Fig. 1 is a view explaining the configuration of the suture thread with needle. The suture thread with needle A which is shown is configured by joining the eyeless needle 1 and the multifilament thread 2.

The eyeless needle 1 is formed with a needlepoint part, (not shown) a middle part 1a continuously formed from the needlepoint part, and the original end face 1b formed at the end of the middle part 1a. Further, a hole 1c having a predetermined diameter and depth is formed along the center of axle from the original end face 1b, and the part corresponding to the hole 1c is formed as the caulked part 1d.

The multifilament thread 2 is configured by a knitted thread or a twisted thread, and includes a thread part 2a having the original thickness and a reduced diameter part 2b, in which one part of the fiber is removed, formed at the end of the thread part 2a. The reduced diameter part 2b is inserted into the hole 1c of the eyeless needle 1 and the caulked part 1d is caulked thereby joining the eyeless needle 1 and the multifilament thread 2. The suture thread with needle A is thus configured.

In the present embodiment, the thickness of the middle part 1a of the eyeless needle 1 is set to 0.43 mm, and the diameter of the hole 1c is set to 0.26 mm (wall thickness is about 0.08 mm). The multifilament thread 2 employs of size 3 to 0, and the thickness of the thread part 2a is set to 0.25 mm. The reduced diameter part 2b of the multifilament thread 2 is set to 0.2 mm so as to have the thickness equal to size 4 to 0 in correspondence to the diameter of the hole 1c.

The reduced diameter part 2b of the multifilament thread 2 is inserted into the hole 1c formed in the eyeless needle 1 and the original end face 1b side is caulked to form the caulked part 1d in correspondence to the depth of the hole 1c, thereby joining the multifilament thread 2 with the eyeless needle 1 to configure the suture thread with needle A.

The thickness of the caulked part 1d in the eyeless needle 1 is defined by the caulking die used when caulking. A plurality of types of caulking die is set in correspondence to the thickness of the middle part 1a of the eyeless needle 1. When the eyeless needle 1 having a thickness of 0.43 mm according to the present embodiment is caulked, the thickness of the caulked part 1d is set so as to be 0.34 mm.

The reduced diameter dimension in the caulked part 1d is, assuming the pull force applied to when suturing or ligaturing the affected area with the suture thread with needle A, set to a value that exhibits the holding force which prevents separation when the pull force is applied to the multifilament thread 2.

Normally, the multifilament thread of size 4 to 0 is applied to the eyeless needle of thickness 0.43 mm. Therefore, with the suture thread with needle in which the thickness of the caulked part of when joining the eyeless needle having the thickness of 0.43 mm and the multifilament thread having the thickness of size 4 to 0 of 0.2 mm is set to 0.34 mm, a difference of 0.14 mm is created between the two.

In the present embodiment, by applying the multifilament thread 1 of size 3 to 0 to the eyeless needle 1 having a thickness of 0.43 mm, the difference between the thickness (0.34 mm) of the original end of the eyeless needle 1 and the thickness (0.25 mm) of the thread part 2a of the multifilament thread 2 of the suture thread with needle A becomes 0.09 mm. That is, compared to the difference of the suture thread with needle which is used in a normal manner, the difference of 0.05 mm is eliminated.

Therefore, when the affected area where elastic force cannot be expected is sutured or ligatured, the blood or body fluid is prevented from exuding out from the site where the eyeless needle 1 has passed.

### [Embodiment 2]

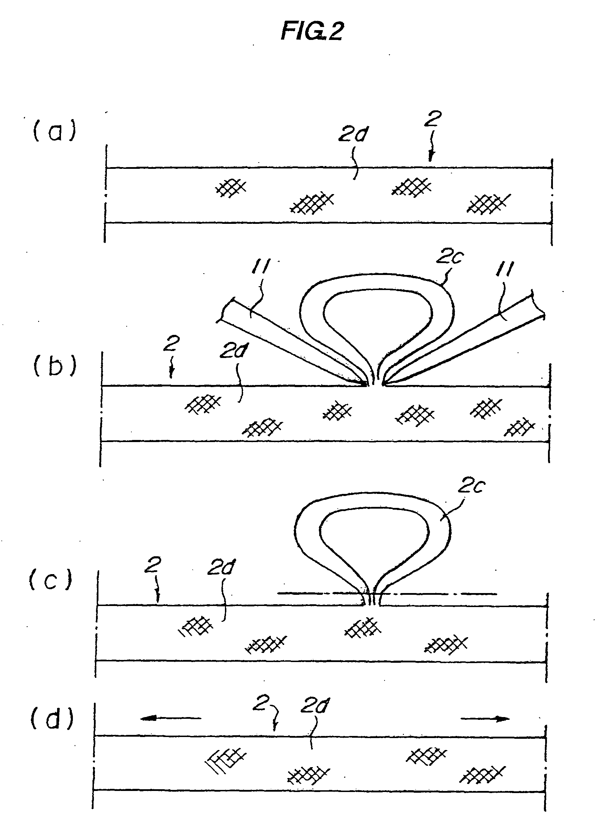

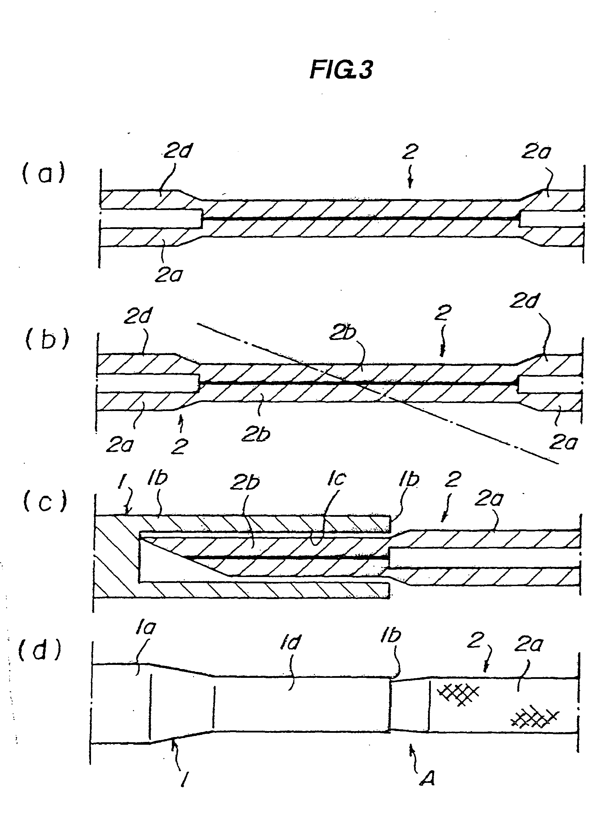

A preferred method of manufacturing the suture thread with needle A according to the present invention will now be explained using the figures. Fig. 2 is a view explaining the procedures for removing one part of the fiber from the multifilament thread. Fig. 3 is a view explaining the procedures for bonding the multifilament thread, in which one part of the fiber is removed, to the hole of the eyeless needle. In the present embodiment, the same components or the components having the same function as those of the previous embodiment are denoted with the same reference signs and thus the explanation thereof is not described.

The procedures for removing the fiber of one part of the multifilament thread 2 will first be explained with Fig. 2. The multifilament thread 2 shown is configured as a knitted thread in which the thin fibers are knitted, and a core thread 2c (refer to Fig. 2B) is arranged at the center. That is, the multifilament thread 2 is formed by covering the periphery of the core thread 2c with the knitted part 2d.

The multifilament thread 2 is cut to a length of approximately twice the length of the multifilament thread 2 configuring the target suture thread with needle A, and the part substantially at the middle part in the longitudinal direction of the cut multifilament thread 2 is straightened (refer to Fig. 2A).

As shown in Fig. 2B, the fiber configuring the knitted part 2d of the multifilament 2 is widened using a needle shaped tool 11, and tweezers and the like (not shown) is inserted from the widened gap to pull out the core thread 2c. It is to be noted that the length of the core thread 2c that is to be pulled out is preferably about twice the length of the reduced diameter part 2b or greater (e.g., about 5 mm to 10 mm) than such value.

Thereafter, as shown in Fig. 2C, the pulled out core thread 2c is cut and removed. Thus, one part of the fiber is removed from the multifilament thread 2. Next, as shown in Fig. 2D, the knitted part 2d is elongated by applying pull force on the multifilament thread 2.

Through the above procedures, the multifilament thread 2 in which one part of the fiber is removed is prepared.

The procedures for configuring the suture thread with needle A by bonding the multifilament thread 2 in which one part of fiber is removed to the eyeless needle 1 will now be explained with Fig. 3. As shown in Fig. 3D, by applying pull force to the multifilament thread 2, the part where the core thread 2c is removed is diameter reduced and such part is applied with adhesive and hardened.

As shown in Fig. 3B, the hardened reduced diameter part is cut diagonally at substantially the middle, thereby configuring two multifilament 2 each having a reduced diameter part 2b, hardened by the adhesive, on the ends. Thus, by forming the reduced diameter part 2b applied with adhesive and hardened in advance, the task of inserting the reduced diameter part 2b into the hole 1c of the eyeless needle 1 in the post to process is easily implemented.

As shown in Fig. 3C, the reduced diameter part 2b is inserted with the multifilament thread 2 facing the hole 1c of the eyeless needle 1. Subsequently, as shown in Fig. 2D, by caulking the part corresponding to the hole 1c using a predetermined caulking die, the eyeless needle 1 and the multifilament thread 2 are joined thereby configuring the suture thread with needle A.

### [Embodiment 3]

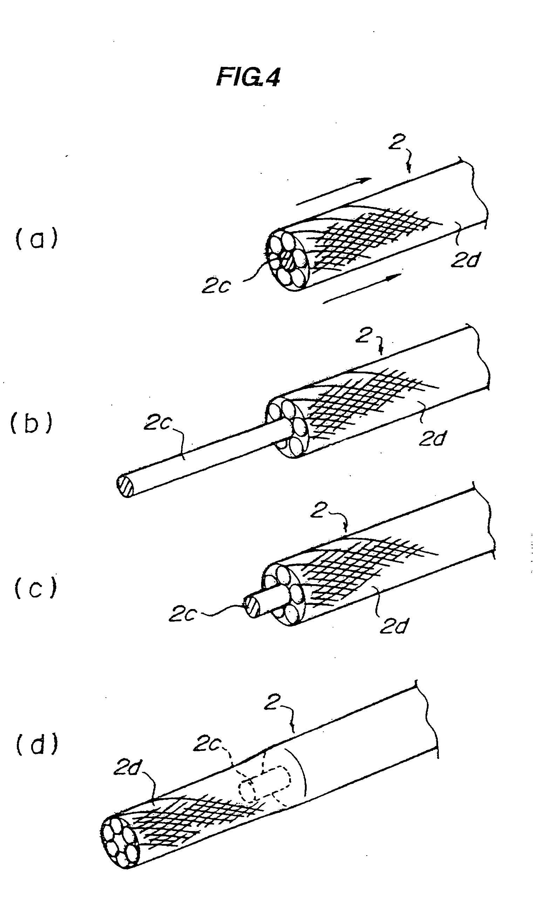

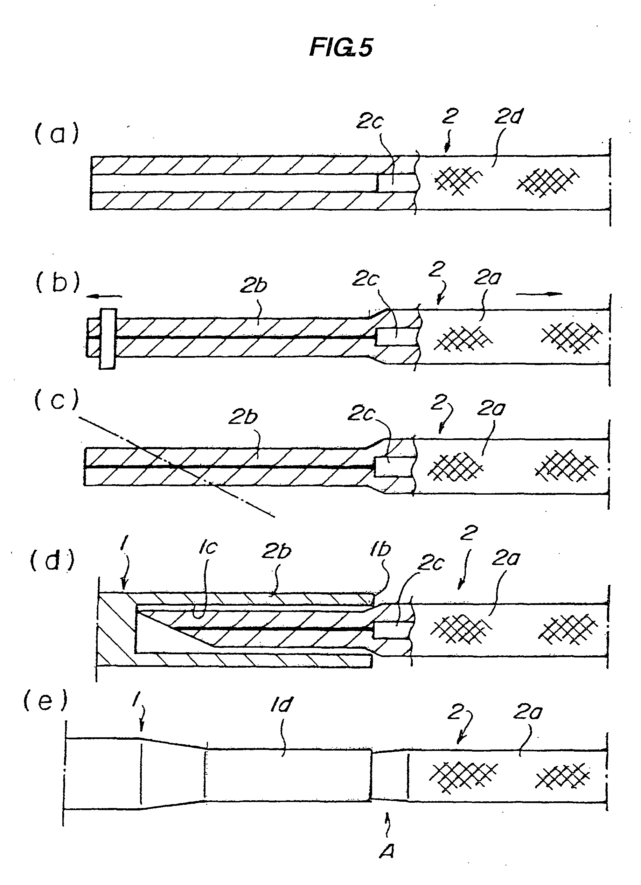

An example of another method for manufacturing the suture thread with needle A according to the present invention will now be explained using the figure. Fig. 4 is a view explaining the procedures for removing one part of the fiber from the multifilament thread. Fig. 5 is a view explaining the procedures for joining the multifilament thread in which one part of the fiber is removed to the hole of the eyeless needle. The multifilament thread 2 which is shown is configured as a knitted thread formed by covering the periphery of the core thread 2c with the knitted part 2d.

In this embodiment, the multifilament thread 2 is cut to a length substantially equal to the length of the multifilament 2 configuring the target suture thread with needle A in advance, and the reduced diameter part 2b is formed at the end of the multifilament thread 2.

As shown in Fig. 4(a), a force in the direction of the middle side of the multifilament thread 2 is applied to the knitted part 2d at the cross section of the end of the multifilament thread 2 to bring the knitted part 2d closer to the middle side. As a result, the core thread 2c is relatively projected outward, as shown in fig. 4B. One part of the fiber is removed by cutting the projected part to a predetermined length (length taking into consideration the grasping margin for grasping in the adhesive application step to be hereinafter described and the cutting margin in addition to the depth of the hole 1c of the eyeless needle 1) (refer to Fig. 4C).

Thereafter, the end of the multifilament thread 2 is worked to return the knitted part 2d that has been brought closer to the middle side to its initial state. Thus, a part in which the core thread 2c is removed is formed at the end of the multifilament thread 2. That is, the fiber of one part of the multifilament thread 2 is removed.

The procedures for joining the above described multifilament thread 2 to the eyeless needle 1 will now be explained with Fig. 5. As shown in Fig. 5A, the core thread 2c is removed from the end of the multifilament thread 2 through the procedures shown in Fig. 4.

As shown in Fig. 5B, when the free end of the knitted part 2d of the multifilament thread 2 is grasped and elongated through application of the pull force, the thickness of the knitted part 2d is reduced and thus has a reduced diameter since the core thread 2c is removed. By applying and hardening the adhesive to the knitted part 2d while maintaining the above state, the reduced diameter part 2b is formed at the end of the multifilament thread 2. After the reduced diameter part 2b is sufficiently hardened, the thread is cut diagonally, as shown in Fig. 5(c).

As shown in Fig. 5D, the reduced diameter part 2d is inserted with the multifilament thread 2 facing the hole 1c of the eyeless needle 1. Subsequently, as shown in Fig. 5E, the part corresponding to the hole 1c is caulked using the predetermined caulking die thereby joining the eyeless needle 1 and the multifilament thread 2 to configure the suture thread with needle A.

In each of the above embodiments, a case in which the multifilament thread 2 having the core thread 2c is explained, but when using the multifilament thread 2 without the core thread 2c, the fiber of the outer peripheral part of the location corresponding to the reduced diameter part 2b or the internal fiber of the multifilament thread 2 is cut with a cutting tool, such as a knife and picked up with tweezers and the like to remove one part of the fiber. In this case, the cut part of the fiber is preferably hardened sufficiently with the adhesive so as not to be separated.

The suture thread with needle A of the present invention has the difference between the caulked part 1d of the eyeless needle 1 and the thread part 2a of the multifilament thread 2 reduced as described above, and thus the multifilament 2 is able to smoothly pass through the body tissue after the eyeless needle 1 has passed. Further, since the difference between the caulked part 1d and the multifilament thread 2 is small, the blood is prevented from exuding out from the site where the eyeless needle 1 has passed even when ligaturing the blood vessel. Thus, the suture thread with needle is extremely effectively used when suturing or ligaturing the body tissue.

## Claims

1. A suture thread with needle configured by caulking and joining a suture thread made of multifilament thread and an eyeless needle,
wherein one part of the fiber at the joining part of the multifilament thread caulked and joined to the eyeless needle is removed, so that the thickness of one part of the multifilament thread is smaller than the thickness of the other part.

2. The suture thread with needle according to claim 1,
the fiber of the multifilament thread which is partly removed at the joining part is a core thread which is disposed through a center of the multifilament thread.

3. The suture thread with needle according to claim 1,
the joining part of the multifilament thread is hardened with adhesive.

4. The suture thread with needle according to claim 3,
the joining part of the multifilament thread is formed by cutting diagonally and inserted into a hole of the eyeless needle
